# EUROPEAN PATENT APPLICATION

(11) **EP 3 806 037 A1**
(43) Date of publication of application: **14.04.2021**
(21) Application number: 19202520.3
(22) Date of filing: 10.10.2019
(51) Int. Cl.: G06T 7/33

(54) **SYSTEM AND CORRESPONDING METHOD AND COMPUTER PROGRAM AND APPARATUS AND CORRESPONDING METHOD AND COMPUTER PROGRAM**

(71) Applicant: Leica Instruments (Singapore) Pte. Ltd., 608924 Singapore (SG)
(72) Inventor: THEMELIS, George, 88131 Lindau (DE)
(74) Representative: 2SPL Patentanwälte PartG mbB

(57) **Abstract**

Examples relate to a system, a method and a computer program for processing a pre-operative three-dimensional scan of a part of a body, to an apparatus, method and computer program for providing a control signal to an image guided system, and to an image guided system comprising such an apparatus. The system for processing a pre-operative three-dimensional scan of a part of a body comprises one or more processors and one or more storage devices. The system is configured to obtain a pre-operative three-dimensional scan of the part of the body. The system is configured to identify anatomical features within the pre-operative three-dimensional scan of the part of the body. The anatomical features are features that are visible in an optical image of the part of the body. The system is configured to provide information on the anatomical features based on the anatomical features identified within the pre-operative three-dimensional scan of the part of the body.

## Description

### Technical field

Examples relate to a system, a method and a computer program for processing a pre-operative three-dimensional scan of a part of a body, to an apparatus, method and computer program for providing a control signal to an image guided system, and to an image guided system comprising such an apparatus.

### Background

Image Guided Systems (IGS) are routinely used as an add-on to surgical microscopes to assist surgeons navigate within the tissue volume, and reach pathologic tissue areas, while avoiding hurting sensitive but vital structures such as brain and vessels. IGSs are utilizing pre-operative three-dimensional scans of the patient, such as MRI (Magnetic Resonance Imaging) scans and X-ray angiography scans. The three-dimensional scans are presented in an augmented reality manner merged with the real time optical image of the human body. Thereby the surgeon may easier and safer perform the surgical procedure. To align the three-dimensional scan with the camera image, a so-called spatial registration of the pre-operative data to the microscope's camera images may be performed. The spatial registration may be performed using rigid features, such as the skull, which is used as a reference, which may provide a reliable spatial registration until the skull is opened and the brain tissue is deformed. A further factor when performing the spatial registration is the performance of the alignment - as a three-dimensional scan contains a vast amount of data regarding various vessels, bones and tissue of the body, in many cases the alignment consumes a high amount of computing resources, which may inhibit a realignment during surgery.

### Summary

There may be a desire for providing an improved approach suitable for aligning a camera image with a three-dimensional pre-operative scan in an image guided system.

The desire is addressed by embodiments of the present disclosure.

Embodiments of the present disclosure provide a system comprising one or more processors and one or more storage devices. The system is configured to obtain a pre-operative three-dimensional scan of a part of a body. The system is configured to identify anatomical features within the pre-operative three-dimensional scan of the part of the body. The anatomical features are features that are visible in an optical image of the part of the body. The system is configured to provide information on the anatomical features based on the anatomical features identified within the pre-operative three-dimensional scan of the part of the body.

Embodiments further provide a corresponding method for processing a pre-operative three-dimensional scan of a part of a body. The method comprises identifying anatomical features within the pre-operative three-dimensional scan of the part of the body. The anatomical features are features that are visible in an optical image of the part of the body. The method comprises providing information on the anatomical features based on the anatomical features identified within the pre-operative three-dimensional scan of the part of the body. Embodiments provide a computer program with a program code for performing the method when the computer program is executed on a processor.

By identifying the anatomical features within the pre-operative three-dimensional scan, the information on the anatomical features may be generated, which may comprise the anatomical features in isolated form. Based on said information on the anatomical features, an alignment between the pre-operative three-dimensional scan and the optical image may be facilitated, as fewer features are to be tracked and matched to corresponding features in an optical image being used at an image-guided system.

Embodiments of the present disclosure further provide an apparatus for providing a control signal to an image guided system. The apparatus comprises an interface for providing the control signal to the image guided system and for obtaining an optical image of a part of a body. The apparatus comprises a processor configured to obtain information on anatomical features. The information on the anatomical features is based on anatomical features identified within a pre-operative three-dimensional scan of a part of a body. The processor is configured to generate the control signal based on a match between the anatomical features of the information on the anatomical features and corresponding anatomical features visible within the optical image of the part of the body. The control signal indicates an alignment between the optical image and the pre-operative three-dimensional scan. The processor is configured to provide the control signal to the image guided system via the interface.

Embodiments of the present disclosure provide a corresponding method for providing a control signal to an image guided system. The method comprises obtaining information on anatomical features. The information on the anatomical features is based on anatomical features identified within a pre-operative three-dimensional scan of a part of a body. The method comprises obtaining an optical image of the part of the body. The method comprises generating the control signal based on a match between the anatomical features of the information on the anatomical features and corresponding anatomical features visible within the optical image of the part of the body. The control signal indicates an alignment between the optical image and the pre-operative three-dimensional scan. The method comprises providing the control signal to the image guided system via the interface. Embodiments further provide a computer program with a program code for performing the method when the computer program is executed on a processor.

By using the information on the anatomical features, in which the anatomical features are identified, instead of the entire pre-operative three-dimensional scan, an alignment between the pre-operative three-dimensional scan and the optical image may be facilitated, as fewer features are to be tracked and matched to corresponding features in the optical image.

### Short description of the Figures

Some examples of apparatuses and/or methods will be described in the following by way of example only, and with reference to the accompanying figures, in which
- Fig. 1a: shows a block diagram of an embodiment of a system for processing a pre-operative three-dimensional scan of a part of a body;
- Fig. 1b: shows a flow chart of an embodiment of a method for processing a pre-operative three-dimensional scan of a part of a body;
- Fig. 2a: shows a block diagram of an embodiment of an apparatus for providing a control signal to an image guided system;
- Fig. 2b: shows a flow chart of an embodiment of a method for providing a control signal to an image guided system;
- Fig. 2c: show a block diagram of the image guided system comprising an apparatus for providing a control signal to an image guided system;
- Fig. 3: shows a block diagram of an example of a system suitable for aligning pre-operative three-dimensional data with an image guided system; and
- Fig. 4: shows a schematic diagram of a system comprising a microscope or an image-guided system and a computer system.

### Detailed Description

Various examples will now be described more fully with reference to the accompanying drawings in which some examples are illustrated. In the figures, the thicknesses of lines, layers and/or regions may be exaggerated for clarity.

Accordingly, while further examples are capable of various modifications and alternative forms, some particular examples thereof are shown in the figures and will subsequently be described in detail. However, this detailed description does not limit further examples to the particular forms described. Further examples may cover all modifications, equivalents, and alternatives falling within the scope of the disclosure. Same or like numbers refer to like or similar elements throughout the description of the figures, which may be implemented identically or in modified form when compared to one another while providing for the same or a similar functionality.

It will be understood that when an element is referred to as being "connected" or "coupled" to another element, the elements may be directly connected or coupled or via one or more intervening elements. If two elements A and B are combined using an "or", this is to be understood to disclose all possible combinations, i.e. only A, only B as well as A and B, if not explicitly or implicitly defined otherwise. An alternative wording for the same combinations is "at least one of A and B" or "A and/or B". The same applies, mutatis mutandis, for combinations of more than two Elements.

The terminology used herein for the purpose of describing particular examples is not intended to be limiting for further examples. Whenever a singular form such as "a," "an" and "the" is used and using only a single element is neither explicitly or implicitly defined as being mandatory, further examples may also use plural elements to implement the same functionality. Likewise, when a functionality is subsequently described as being implemented using multiple elements, further examples may implement the same functionality using a single element or processing entity. It will be further understood that the terms "comprises," "comprising," "includes" and/or "including," when used, specify the presence of the stated features, integers, steps, operations, processes, acts, elements and/or components, but do not preclude the presence or addition of one or more other features, integers, steps, operations, processes, acts, elements, components and/or any group thereof.

Unless otherwise defined, all terms (including technical and scientific terms) are used herein in their ordinary meaning of the art to which the examples belong.

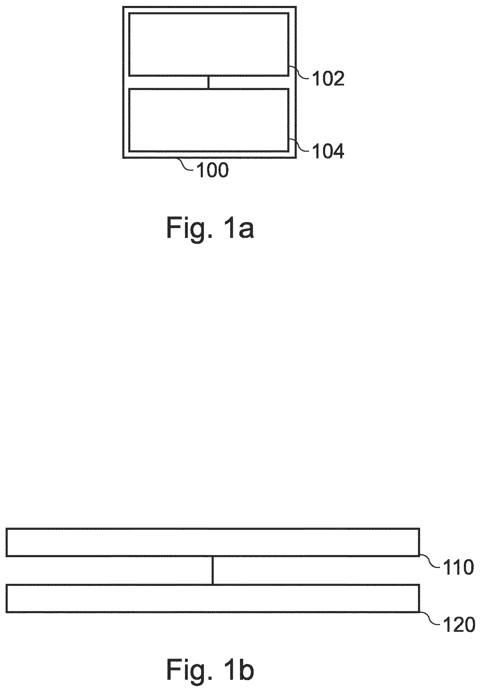

Fig. 1a shows a block diagram of an embodiment of a system 100 comprising one or more processors 102 and one or more storage devices 104, the one or more storage devices 104 being coupled to the one or more processors. The system is suitable for (pre-) processing a pre-operative three-dimensional scan of a part of a body. The system is configured to obtain the pre-operative three-dimensional scan of the part of the body. The system is configured to identify anatomical features within the pre-operative three-dimensional scan of the part of the body. The anatomical features are features that are visible in an optical image of the part of the body. The system is configured to provide information on the anatomical features based on the anatomical features identified within the pre-operative three-dimensional scan of the part of the body.

Fig. 1b shows a flow chart of a corresponding method, e.g. of a corresponding computer-implemented method. The method is suitable for processing the pre-operative three-dimensional scan of the part of the body. The method comprises Identifying 110 the anatomical features within the pre-operative three-dimensional scan of the part of the body. The method comprises providing the information on the anatomical features based on the anatomical features identified within the pre-operative three-dimensional scan of the part of the body. For example, the method may be performed or executed by the system of Fig. 1a.

The following description relates both to the system of Fig. 1a and to the corresponding method of Fig. 1b.

Embodiments provide a system, method and computer program suitable for processing a pre-operative three-dimensional scan. Such pre-operative three-dimensional scans are often used for alignment in an image-guided system. In image guided systems, the pre-operative three-dimensional scan may be used to guide the surgeon during surgery, e.g. by enabling the surgeon to see the three-dimensional representation of the part of the body as he or she performs the surgery. In many cases, the pre-operative three-dimensional scan is overlaid over an optical image provided by the camera, e.g. in an augmented reality manner, to provide the surgeon with additional information about the part of the body that is currently visible through the microscope.

In embodiments, in order to facilitate the alignment, the pre-operative three-dimensional scan is (pre-)processed before it is used for the alignment. One aspect of the pre-processing is the identification of suitable anatomical features within the pre-operative three-dimensional scan that can also be seen in the optical image. The system is thus configured to identify the (suitable) anatomical features within the pre-operative three-dimensional scan of the part of the body. In this context, the term "suitability" may refer to the visibility of the respective anatomical feature within the optical image. "Suitable" anatomical features may be anatomical features that are (clearly) distinguishable within the optical image. For example, the three-dimensional scan of the part of the body may be magnetic resonance imaging scan (an MRI scan) or an X-ray angiography scan of the part of the body. In some embodiments, the pre-operative three-dimensional scan may be limited to the part of the body. Alternatively, the pre-operative three-dimensional scan may comprise three-dimensional data of other parts of the body in addition to the three-dimensional data of the part of the body. In some embodiments, e.g. in embodiments relate to brain surgery, the part of the body may be the head or brain. For example, the pre-operative three-dimensional scan may be obtained from a three-dimensional scanning device, such as an MRI or an X-ray angiography device. In at least some embodiments, the pre-operative three-dimensional scan may be obtained from one of the one or more storage devices 104.

In at least some embodiments, the anatomical features are non-rigid anatomical features. For example, at least a subset of the anatomical features may be non-rigid anatomical features. In this context, the term "non-rigid" may be interpreted as "unfixed", "movable" or "movable during surgery", e.g. relative to a reference position in the optical image. For example, the anatomical features may be non-rigid anatomical features in close proximity to a portion of the part of the body on which the surgery is performed. During the surgery, the anatomical features may be moved, e.g. to enable the surgeon to perform the surgery. In other words, the anatomical features may be anatomical features of soft tissue (or of one or more bones) to be moved in a surgical procedure. For example, the anatomical features may comprise one or more elements of the group of one or more vessels (such as one or more arteries or one or more veins), one or more bones and one or more tumors. In this context, some bones may be considered non-rigid, e.g. if the bones are moveable during the surgical procedure. Some other bones, e.g. the skull, if the surgical procedure is a brain surgical procedure, may be considered as rigid anatomical structures, unsuitable for use with embodiments.

The anatomical features are visible in an optical image of the part of the body. In this context, the term "visible in an optical image" may be interpreted as at least a part of an anatomical feature being distinguishable within an optical image taken of the part of the body. To be visible, the anatomical features may be located at or close to the surface of the part of the body, and provide at least a minimal contrast to adjacent portions of the part of the body.

The anatomical features are identified within the pre-operative three-dimensional scan. In embodiments, the identification of the anatomical features may be performed using object recognition (e.g. using a machine-learning model or using a comparison with known shapes of suitable anatomical features), or using manual annotation. For example, using object recognition and/or using machine learning, different categories (such as the categories of "blood vessels", "bones" and "tumors") of anatomical features may be detected within the pre-operative three-dimensional scan to identify the anatomical features within the pre-operative three-dimensional scan. For example, the object recognition may be implemented using the machine-learning model. The machine-learning model may be used to identify the different categories of anatomical features within the pre-operative three-dimensional scan. The machine-learning model may be trained using training samples that comprise three-dimensional image data of suitable anatomical features, and a desired output, in which said suitable anatomical features are highlighted or isolated. For example, different machine-learning models may be used for each category of anatomical features. The different machine-learning models may be trained accordingly, e.g. using a desired output in which (only) suitable anatomical features of the respective category are highlighted or isolated. Within the information on the anatomical features, the anatomical features may be represented according to the different categories of anatomical features. For example, the information on the anatomical features may comprise information on the category of the respective anatomical feature for each anatomical featured represented by the information on the anatomical features. Alternatively or additionally, the information on the anatomical features may be sub-divided by the different categories.

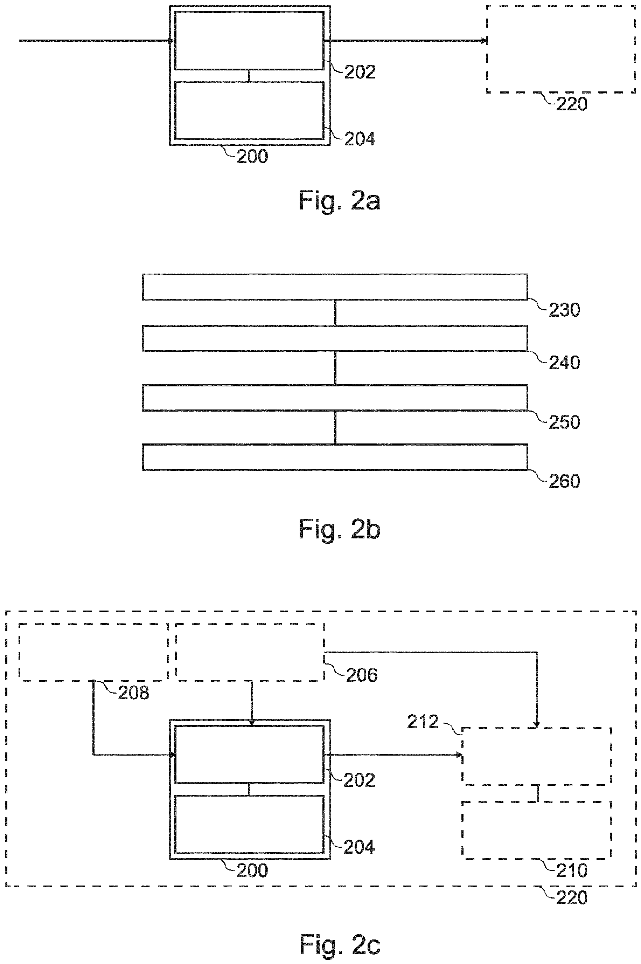

The system is configured to provide the information on the anatomical features based on the anatomical features identified within the pre-operative three-dimensional scan of the part of the body. For example, the system may be configured to store the information on the anatomical features using one of the one or more storage devices 104 or one or more storage devices external to the system, e.g. to a storage device of the apparatus 200 of Fig. 2a. For example, the system may be configured to provide the information on the anatomical features to the apparatus 200 of Fig. 2. For example, the information on the anatomical features may be saved within one or more files or within a database structure on a storage device.

In at least some embodiments, the information on the anatomical features is based on the pre-operative three-dimensional scan, e.g. a thinned-out version of the pre-operative three-dimensional scan, with tissue, vessels and/or bones being removed apart from the anatomical features. In some embodiments, the information on the anatomical features might (only) comprise the anatomical features. Other features present within the pre-operative three-dimensional scan may be suppressed within the information on the anatomical features. Accordingly, the system may be configured to isolate the anatomical features within the pre-operative three-dimensional scan, and to use the isolated features for the information on the anatomical features. For example, the anatomical features may be isolated within the pre-operative three-dimensional scan (and, consequently, the information on the anatomical features) by removing or hiding (all) other features from the pre-operative three-dimensional scan (e.g. for the purpose of generating the control signal), by highlighting the anatomical features within the pre-operative three-dimensional scan, or by extracting the anatomical features from the pre-operative three-dimensional scan, thereby creating a new pre-operative three-dimensional scan as the information on the anatomical features. By isolating the anatomical features, a matching of features between the optical image and the pre-operative three-dimensional scan may be improved in both accuracy and speed, as fewer features may be matched. For example, the anatomical features may be identified and isolated within the pre-operative three-dimensional scan before surgery, e.g. as a pre-processing step, resulting in the information on the anatomical features.

Alternatively, the information on the anatomical features may be an annotated version of the pre-operative three-dimensional scan, e.g. with the anatomical features being marked, highlighted or annotated within the information on the anatomical features, e.g. with the marks, highlights and/or annotations being based on the identification of the anatomical features. In some embodiments, the information on the anatomical features may comprise (only) the annotations, the annotations comprising the identification of the anatomical features, to be used in conjunction with the pre-operative three-dimensional scan.

To further improve an accuracy of the alignment between the pre-operative three-dimensional scan and the optical image, in addition to the anatomical features, a surface structure of the part of the body may be used. Accordingly, in some embodiments, the system may be configured to determine a surface structure of tissue of the part of the body based on the pre-operative three-dimensional scan. For example, the system may be configured to extract the surface structure of the tissue of the part of the body based on the pre-operative three-dimensional scan. The system may be further configured to provide information on the surface structure of the tissue of the part of the body. For example, the information on the surface structure may be provided similar to, or with, the information on the anatomical features.

In general, the one or more processors and one or more storage devices may be part of a local computing device or part of a distributed system. In embodiments the one or more processors 102 may be implemented using one or more processing units, one or more processing devices, any means for processing, such as a processor, a computer or a programmable hardware component being operable with accordingly adapted software. In other words, the described function of the one or more processors 102 may as well be implemented in software, which is then executed on one or more programmable hardware components. Such hardware components may comprise a general purpose processor, a Digital Signal Processor (DSP), a micro-controller, etc. The one or more storage devices 104 may comprise any kind of memory or storage circuitry, the circuitry being suitable for digitally storing information. In at least some embodiments, the one or more storage devices 104 may comprise at least one element of the group of a computer readable storage medium, such as an magnetic or optical storage medium, e.g. a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage. In at least some embodiments, the system may further comprise at least one interface for communicating with other devices, apparatuses or entities. For example, the at least one interface may correspond to one or more inputs and/or outputs for receiving and/or transmitting information, which may be in digital (bit) values according to a specified code, within a module, between modules or between modules of different entities. For example, the at least one interface may comprise interface circuitry configured to receive and/or transmit information.

More details and aspects of the system, method and computer program are mentioned in connection with the proposed concept or one or more examples described above or below (e.g. Fig. 2a to 3). The system, method and computer program may comprise one or more additional optional features corresponding to one or more aspects of the proposed concept or one or more examples described above or below.

Embodiments further provide a method, apparatus and computer program for providing a control signal to an image guided system.

Fig. 2a shows a block diagram of an apparatus 200 for providing a control signal to an image guided system. The apparatus 200 comprises an interface 202 for providing the control signal to the image guided system and for obtaining an optical image of a part of a body. The apparatus 200 further comprises a processor 204 that is coupled to the interface 202. The processor 204 is configured to obtain information on anatomical features (e.g. the information on the anatomical features provided by the system of Fig. 1a). The information on the anatomical features is based on anatomical features identified within a pre-operative three-dimensional scan of the part of the body. The processor 204 is configured to generate the control signal based on a match between the anatomical features of the information on the anatomical features and corresponding anatomical features visible within the optical image of the part of the body. The control signal indicates an alignment between the optical image and the pre-operative three-dimensional scan. The processor is configured to provide the control signal to the image guided system via the interface. In some embodiments, the apparatus 200 may further comprise the system 100 of Fig. 1a or be configured to execute the method introduced in connection with Fig. 1b.

Fig. 2b shows a flow chart of an embodiment of a corresponding method for providing a control signal to an image guided system. The method comprises obtaining 230 the information on the anatomical features. The method comprises obtaining 240 the optical image of the part of the body. The method comprises generating 250 the control signal based on the match between the anatomical features of the information on the anatomical features and the corresponding anatomical features visible within the optical image of the part of the body. The method comprises providing 260 the control signal to the image guided system. For example, the method may be executed by the apparatus 200 of Fig. 2a. For example, the method may further comprise the method introduced in connection with Fig. 1b.

The following description relates to the apparatus 200 of Fig. 2a and to the method of Fig. 2b, and, at a later stage, to the image guided system of Fig. 2c.

Embodiments provide an apparatus, method and computer program suitable for providing a control signal to an image guided system. As laid out above, in image guided systems, the pre-operative three-dimensional scan may be used to guide the surgeon during surgery, e.g. by enabling the surgeon to see the three-dimensional representation of the part of the body as he or she performs the surgery. In many cases, the pre-operative three-dimensional scan is overlaid over an optical image provided by the camera, e.g. in an augmented reality manner, to provide the surgeon with additional information about the part of the body that is currently visible through the microscope. In other words, the image guided system, e.g. the image guided system 220 of Fig. 2c, may be suitable for overlaying a visual augmented reality representation of the pre-operative three-dimensional scan over the optical image of the part of the body. For example, the optical image may originate from a camera of a microscope, e.g. a microscope of the image guided system. For example, the optical image may be a still image or part of a video. In some embodiments, the optical image may be a stereoscopic optical image.

The visual augmented reality representation of the pre-operative three-dimensional scan may be aligned with the optical image of the part of the body, e.g. so the features that are visible within the pre-operative three-dimensional scan are overlaid over the correct part of the optical image. Before the operation starts, or as long as soft tissue remains unchanged, such an alignment may be performed using rigid features, such as the skull of the patient. Such an alignment between the pre-operative three-dimensional scan and the optical image may be denoted "spatial registration". For example, the rigid features of the part of the body may be used as a starting point in the alignment between the anatomical features of the information on the anatomical features and the corresponding anatomical features visible within the optical image of the part of the body. After the operation starts, and soft tissue is being moved or deformed, the alignment between the pre-operative three-dimensional scan and the optical image may become more difficult to perform. Embodiments of the present disclosure may enable and/or provide such an alignment, e.g. by using the anatomical features identified and included in the information on the anatomical features, which may be non-rigid anatomical features, such as blood vessels, tumors, bones etc. that are distinguishable both in the pre-operative three-dimensional scan and the optical image, to perform the alignment. As the actual overlay and/or alignment may be performed by the image guided system, embodiments may provide the control signal to the image guided system, which may be used by the image guided system in the alignment between the pre-operative three-dimensional scan and the optical image. Accordingly, the control signal may be suitable for aligning the visual augmented reality representation of the pre-operative three-dimensional scan with the optical image based on the anatomical features. The control signal indicates an alignment between the optical image and the pre-operative three-dimensional scan. In other words, the control signal indicates how the pre-operative three-dimensional scan is to be transformed to be in alignment with the optical image. For example, the control signal may comprise one or more transformation/deformation vectors and/or morphological operators suitable for deforming the pre-operative three-dimensional scan such that it aligns with the optical image of the part of the body.

The processor is configured to obtain (e.g. to receive or read) the information on the anatomical features. For example, the processor may be configured to obtain the information on the anatomical features from a storage device of the apparatus or of the image guided system 220, from a storage device 104 of the system 100 of Fig. 1a, or from a storage device that is external to the image guided system 220. The processor is further configured to obtain the optical image, e.g. from a camera 206 of the image guided system 220, or from any of the above storage devices. In some embodiments, the information on the anatomical features may comprise the pre-operative three-dimensional scan or a thinned-out version of the pre-operative three-dimensional scan. For example, the information may comprise a version of the pre-operative three-dimensional scan, in which the anatomical features are isolated. Alternatively or additionally, the processor may be configured to obtain the pre-operative three-dimensional scan, e.g. as described in connection with the system of Fig. 1a.

The processor is configured to generate the control signal based on a match between the anatomical features of the information on the anatomical features (i.e. the anatomical features comprised in, or represented in, the information on the anatomical features, short the anatomical features of the information on the anatomical features) and corresponding anatomical features visible within the optical image of the part of the body. In this context, a match between two features may correspond to an association between an anatomical feature identified within the pre-operative three-dimensional scan and an anatomical feature visible within the optical image, where both anatomical features relate to the same anatomical feature of the part of the body. In other words, the processor may be configured to match the anatomical features of the information on the anatomical features with the corresponding anatomical features visible within the optical image of the part of the body. For example, not every anatomical feature of the information on anatomical features may be visible within the optical image of the part of the body - some anatomical features may be obstructed by other tissue, blood etc. Accordingly, the match may be performed between the features that are visible within the optical image of the part of the body and the corresponding anatomical features of the information on the anatomical features. Anatomical features of the information on the anatomical features not visible within the optical image may be disregarded. For example, the optical image may be a microscopic image obtained from a camera of a microscope. In other words, the anatomical features may be visible through the microscope. The match between the anatomical features of the information on the anatomical features and the corresponding anatomical features visible within the optical image of the part of the body may be based on a zoom level of the microscope. This may aid the matching or alignment between the pre-operative three-dimensional scan and the optical image.

In at least some embodiments, the processor may be configured to identify anatomical features corresponding to the anatomical features of the information on the anatomical features within the optical image, e.g. by detecting features matching the criteria of the anatomical features. The anatomical features that are detected within the optical image may be matched to the corresponding anatomical features of the information on the anatomical features, e.g. based on a location of the anatomical features within the optical image. Once the anatomical features are matched between the pre-operative three-dimensional scan and the optical image, the pre-operative three-dimensional scan and the optical image may be aligned. In other words, the processor may be configured to align the pre-operative three-dimensional scan and the optical image, e.g. by transforming (i.e. deforming) the pre-operative three-dimensional scan so it matches the optical image. To enable or improve a comparison of the location of the anatomical features (of the information on the anatomical features) within the pre-operative three-dimensional scan and the optical image, the anatomical features of the information on the anatomical features may be projected (based on their locations within the pre-operative three-dimensional scan) onto a two-dimensional plane that matches an angle of view of a microscope supplying the optical image. In other words, the processor may be configured to project the anatomical features of the information on the anatomical features (and their positions within the pre-operative three-dimensional scan) onto a two-dimensional plane corresponding to a plane of the optical image based on the viewing angle of the microscope providing the optical image. The alignment may be performed by comparing a location of the anatomical features of the information on the anatomical features within the pre-operative three-dimensional scan to a location of the corresponding anatomical features in the optical image, calculating an offset between the two sets of locations, and calculating one or more transformation/deformation vectors or morphological operators to be applied to the pre-operative three-dimensional scan in order to align the pre-operative three-dimensional scan to the optical image.

For example, the pre-operative three-dimensional scan may be deformed in a physically correct manner based on the one or more transformation/deformation vectors or morphological operators, so that tissue or features located adjacent to the anatomical features are transformed/deformed in accordance with the transformation and/or deformation of the anatomical features. The control signal may be generated based on the alignment between the anatomical features of the information on the anatomical features (and their positions within the pre-operative three-dimensional scan) and the anatomical features identified within the optical image.

To improve the detection and/or identification of the anatomical features within the optical image, a multi-spectral optical image may be used, i.e. an image that comprises multiple sub-images taken in different spectral bands (e.g. in addition or instead of red, green and blue spectral bands). In other words, the optical image may be a multi-spectral optical image. The multi-spectral optical image may comprise one or more sub-images taken at spectral bands that are indicative of at least a subset of the anatomical features, e.g. in which the anatomical features, such as blood vessels, bones or tumors, are better distinguishable. For example, one or more spectral bands of the multi-spectral bands may be chosen based on a reflectance, fluorescence and/or bio-luminescence of the anatomical features. The anatomical features may be identified within the optical image using two more spectral bands (e.g. two or more spectral bands that are indicative of at least a subset of the anatomical features) of the multi-spectral optical image.

In some embodiments, a machine-learning model is used to identify different categories of anatomical features within the optical image, e.g. similar to the detection and/or identification of the different categories of anatomical features within the pre-operative three-dimensional scan. This may enable or improve the detection of the anatomical features within the optical image without requiring manual annotation of the optical image. The machine-learning model may be implemented similar to the one introduced in connection with the detection of the anatomical features within the pre-operative three-dimensional scan. For example, in this case, the machine-learning model may be trained using training samples that comprise optical images (e.g. multi-spectral optical images) comprising suitable anatomical features, and a desired output, in which said suitable anatomical features are highlighted or isolated. For example, multiple machine-learning models may be used, one for each category of anatomical features, or a common machine-learning models that is suitable for (all) categories of anatomical features. The control signal may be generated based on an alignment between the anatomical features of the information on the anatomical features of a category of the different categories of anatomical features and the anatomical features of the category identified within the optical image.

To further improve an accuracy of the alignment or match between the pre-operative three-dimensional scan and the optical image, in addition to the anatomical features, a surface structure of the part of the body may be used. In other words, the processor may be configured to obtain information on a surface structure of tissue of the part of the body that is based on the pre-operative three-dimensional scan, e.g. from the system 100 of Fig. 1a. The control signal may be generated based on a match and/or an alignment between the obtained information on to the surface structure and a corresponding surface structure visible with-in the optical image. To align or match the surface structures, the surface structure of the tissue of the part of the body may be identified within the optical image. Accordingly, the processor may be configured to identify the surface structure of the tissue of the part of the body within the optical image, e.g. using a surface or pattern matching technique. The control signal may be generated based on an alignment between the obtained information on the surface structure and the surface structure identified within the optical image.

In order to further improve the alignment, or instead of matching the surface visible within the optical image and the surface structure using the optical image, a depth image of the part of the body may be used. The depth image may be aligned with the optical image, e.g. the depth image may be in a fixed positional and direction relationship with the optical image. The processor may be configured to identify the surface structure of the tissue of the part of the body within a depth image being aligned with the optical image. The control signal may be generated further based on an alignment between the obtained information on the surface structure and the surface structure identified within the depth image (e.g. in addition to the alignment between the anatomical features). For example, the alignment between the obtained information on the surface structure and the surface structure identified within the depth image may be used in addition to, or instead of, an alignment between the obtained information on the surface structure and the surface structure visible within the optical image. For example, the depth image may be one of a Time-of-Flight-based depth image, a structured light-based depth image and a depth image of a stereoscopic camera. For example, the microscope may comprise a stereoscopic camera providing both the depth image and the optical image.

In some cases, the difference between the optical image and the pre-operative three-dimensional scan may become too large, e.g. in case the patient is turned, or in case a portion of the part of the body is removed. To improve the alignment in such cases, an intra-operational (three-dimensional) scan may be used to detect the anatomical features of the information on the anatomical features in the part of the body. For example, the processor may be configured to identify anatomical features corresponding to the anatomical features of information on the anatomical features within an intraoperative scan of the part of the body. The intraoperative scan may have a known orientation (e.g. position and direction) relative to the optical image. For example, the intraoperative scan may be an intraoperative ultrasound scan or an intraoperative X-ray angiography scan of the part of the body, which may be performed during the operation to re-establish the alignment between the optical image and the pre-operative three-dimensional scan. Similar to the detection and identification of the anatomical features in the pre-operative three-dimensional scan and/or the optical image, object recognition and/or a machine-learning model may be used to identify the anatomical features corresponding to the anatomical features of the information on the anatomical features within the intraoperative scan of the part of the body. Furthermore, the control signal may be generated based on an alignment between the anatomical features of the information on the anatomical features and the anatomical features identified within the intraoperative scan of the part of the body.

Each alignment between the optical image and the pre-operative three-dimensional scan may start with an initial alignment, which may be based on a known position of the patient and a known angle of the optical camera. In any case, as the optical image may be supplied by a microscope, which may have a very narrow angle of view, even if the position of the patient and the angle of the camera are known, the initial alignment may be a challenge. In some cases, (only) the anatomical features of the information on the anatomical features and the corresponding anatomical features visible in the optical image may be used for an initial alignment between the anatomical features of the information on the anatomical features and the corresponding anatomical features visible in the optical image. Alternatively, rigid features, such as parts of a skull, may aid the initial alignment. For example, rigid features of the part of the body may be used as a starting point in the alignment between the anatomical features of the information on the anatomical features and the corresponding anatomical features visible within the optical image of the part of the body. For example, the rigid features may be features that have a fixed alignment with the part of the body, i.e. that will be moved less than a pre-defined threshold distance during the operation.

Finally, the processor is configured to provide the control signal to the image guided system. For example, the control signal may be provided as a digital signal, e.g. as network packets or as data written to a memory, or as an analog signal.

In many cases, the part of the body is changed by the surgery, as tissue is moved during surgery. Therefore, instead of having single "alignment operations" to manually reestablish alignment between the pre-operative three-dimensional scan and the optical image, the alignment may be automatically repeated, e.g. periodically or whenever a change within the optical image is larger than a threshold. For example, the optical image may be part of a video stream of the part of the body. In other words, the optical image may be periodically updated as part of the video stream. Accordingly, the control signal may be updated, e.g. periodically or whenever a change within the optical image is larger than a threshold, based on updates to the optical image within the video stream of the part of the body.

Fig. 2c shows a block diagram of the image guided system 220 comprising the apparatus 10. Optionally, the image guided system may comprise a camera 206 for providing the optical image, which may be a video camera, a still-image camera, and/or a stereoscopic camera. In addition, the image guided system may comprise a depth sensor 208, such as a Time of Flight sensor or a Structured Light sensor, for providing the depth image. The image guided system 220 may further comprise a visual output 210, such as a display or a projector, on which the optical image may be displayed with an overlay that is based on the pre-operative three-dimensional scan and the control signal. The visual output 210 may be controlled by a visual controller module 212, which may be configured to merge the optical image with the pre-operative three-dimensional scan based on the control signal. In some embodiments, the image guided system 220 may further comprise the system 100 of Fig. 1a.

The interface 202 may correspond to one or more inputs and/or outputs for receiving and/or transmitting information, which may be in digital (bit) values according to a specified code, within a module, between modules or between modules of different entities. For example, the interface 202 may comprise interface circuitry configured to receive and/or transmit information.

In embodiments the processor 204, and/or the visual controller module 212, may be implemented using one or more processing units, one or more processing devices, any means for processing, such as a processor, processing circuitry, a computer or a programmable hardware component being operable with accordingly adapted software. In other words, the described function of the processing module 204 or of the visual controller module 212 may as well be implemented in software, which is then executed on one or more programmable hardware components. Such hardware components may comprise a general purpose processor, a Digital Signal Processor (DSP), a micro-controller, etc.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

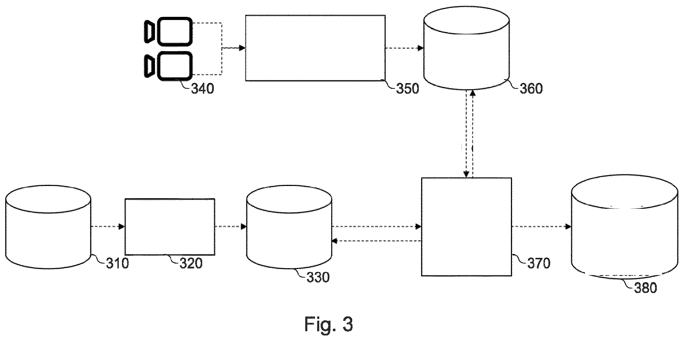

More details and aspects of the apparatus, method and computer program for providing a control signal to an image guided system are mentioned in connection with the proposed concept or one or more examples described above or below (e.g. Fig. 3). The apparatus, method and computer program for providing a control signal to an image guided system may comprise one or more additional optional features corresponding to one or more aspects of the proposed concept or one or more examples described above or below.

Fig. 3 shows a block diagram of an example of a system suitable for aligning pre-operative three-dimensional data with an image guided system. At least some embodiments are based on pre-processing data from pre-operational three-dimensional (3D) data to be used for optical recognition/registration. Accordingly, at least some embodiments relate to a pre-processing of 3D data, e.g. as performed by the system, method and computer program of Figs. 1a and/or 1b.

Embodiments provide a method for improving a spatial registration of pre-operative 3D data (e.g. the pre-operative three-dimensional scan) to a surgical microscope image (e.g. the optical image). To improve the spatial registration, anatomical features of the soft tissue, e.g. veins and arteries, may be used as landmarks as they can be seen in both optical and pre-operative imaging. One limitation in this approach is the processing power required for this process, as a volume scan of the whole brain contains a lot of features. It would be difficult to segment the scan and compare it to the deformed soft tissue of a very thin superficial layer.

Embodiments thus provide a method that may be suitable for making the process or intraoperative registration of pre-operative 3D data to the surgical microscope image more efficient in terms of speed, accuracy and robustness. In order to achieve that, at least some of the following features may be used.

In embodiments, the 3D pre-operative data 310 (e.g. the pre-operative three-dimensional scan) may be processed 320 (e.g. in offline-processing before the surgery) in such a way that anatomical structures (e.g. anatomical features of the information on the anatomical features) that can be recognized on an optical image are segmented and extracted 330 (i.e. isolated) from the 3D volumetric data. Such structures may, for example, include vessels (such as arteries and veins), bones, and tumors. By keeping (only) these anatomical structures, it may become much easier to perform a comparison to the real time optical image.

During surgery, one or more cameras 340 of the microscope of the IGS, which may provide 2D or stereo optical images, may provide the optical images, which may be processed 350 "online" (i.e. during the surgery), and which may yield segmented anatomical structures 360 of anatomical features, at least some of which may correspond to the anatomical features segmented from the pre-operative three-dimensional scan. The segmented anatomical structures 330; 360 may be used in an "online" (i.e. during the surgery) comparison matching 370 of structures (e.g. as performed in the generation of the control signal by the apparatus 200 of Fig. 2a). The relevant data of the segmented anatomical structures 330; 360 may be provided by the respective blocks 330; 360. Optionally, the online comparison matching 370 of structures may request the data of blocks 330; 360. The online comparison matching 370 of structures may yield spatially registered and deformation-corrected anatomical structures, which may correspond to the control signal, or which may be used to generate the control signal.

In some embodiments, multispectral imaging (in the microscopic camera image) may distinguish the arteries and veins, and may therefore improve the correlation to the 3D data where the arteries and veins could be distinguished from the anatomical shape. Thereby, by using this additional information, the accuracy and speed of the alignment may be improved.

During the operation, the system may use a conventional IGS registration system at the first operation steps that can offer good accuracy, so that the auto-registration algorithm will use it as an initial search for spatial correlation instead of starting form "blank paper".

For example, the camera of the microscope 240 can be a 2D camera or a (3D) stereoscopic camera. Stereoscopic images could be further processed to extract a 3D mapping of the tissue surface, which can further improve accuracy.

In at least some embodiments, the optical magnification and working distance of the microscope 240, which are usually known, may allow the calculation of the actual scale of the microscope-captured images. This can be an additional information to be used for the correlation of the data.

An additional 3D scanner such as time flight camera (TOF), or structured light projector may be used to capture the tissue surface 3D profile. This may be useful for the more effective function of the comparison algorithm, especially when some of the tissue is intact and not severely deformed. The brain white matter surface has characteristic surface patterns which may be unique for each person's brain.

In some cases, additional intraoperative use of other modalities such as ultrasound and x-ray angiography may assist the process by offering a 3D view of all the tissue volume. Such a process may be used as a recalibration of the system in cases where the optical images alone fail to reveal the location.

More details and aspects of the present concept are mentioned in connection with the proposed concept or one or more examples described above or below (e.g. Fig. 1a to 2b). The present concept may comprise one or more additional optional features corresponding to one or more aspects of the proposed concept or one or more examples described above or below.

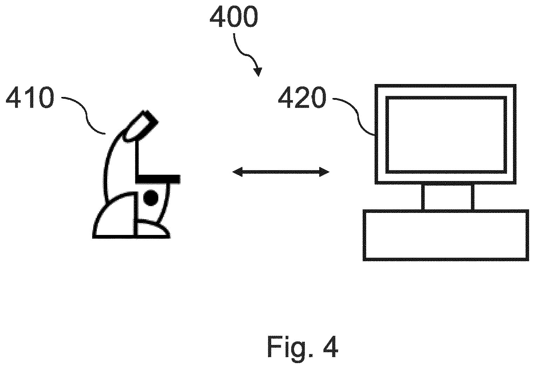

Some embodiments relate to a microscope or image-guided system comprising a system as described in connection with one or more of the Figs. 1 to 3. Alternatively, a microscope or image-guided system may be part of or connected to a system as described in connection with one or more of the Figs. 1 to 3. Fig. 4 shows a schematic illustration of a system 400 configured to perform a method described herein. The system 400 comprises a microscope or image-guided system 410 and a computer system 420. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus. Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### List of reference Signs

- 100: System
- 102: One or more processors
- 104: One or more storage devices
- 110: Identifying anatomical features in a pre-operative three-dimensional scan
- 120: Providing information on the anatomical features
- 200: Apparatus for providing a control signal to an image guided system
- 202: Interface
- 204: Processing module
- 206: Camera
- 208: Depth sensor
- 210: Visual output
- 212: Visual controller module
- 220: Image guided system
- 230: Obtaining information on anatomical features
- 240: Obtaining an optical image
- 250: Generating a control signal
- 260: Providing the control signal
- 310: Pre-operative 3D data
- 320: Offline preprocessing
- 330: Segmented anatomical structures
- 340: Microscope camera(s) 2D or stereo
- 350: Online processing of 2D or stereoscopic optical images
- 360: Segmented anatomical structures
- 370: Online comparison matching of structures
- 380: Spatially registered and deformation-corrected anatomical structures
- 400: System
- 410: Microscope or Image-Guided System
- 420: Computer system

## Claims

1. A system (100) comprising one or more processors (102) and one or more storage devices (104), wherein the system is configured to:
Obtain a pre-operative three-dimensional scan of a part of a body,
Identify anatomical features within the pre-operative three-dimensional scan of the part of the body, the anatomical features being features that are visible in an optical image of the part of the body, and
Provide information on the anatomical features based on the anatomical features identified within the pre-operative three-dimensional scan of the part of the body.

2. The system according to claim 1, wherein at least a subset of the anatomical features are non-rigid anatomical features,
and/or wherein the anatomical features are anatomical features of soft tissue to be moved in a surgical procedure,
and/or wherein the anatomical features comprise one or more elements of the group of one or more arteries, one or more veins, one or more bones and one or more tumors.

3. The system according to one of the claims 1 or 2, wherein a machine-learning model is used to identify different categories of anatomical features within the pre-operative three-dimensional scan, wherein the anatomical features are represented according to the different categories of anatomical features within the information on the anatomical features.

4. The system according to one of the claims 1 to 3, wherein the system is configured to determine a surface structure of tissue of the part of the body based on the pre-operative three-dimensional scan, and to provide information on the surface structure of the tissue of the part of the body.

5. An apparatus (200) for providing a control signal to an image guided system (220; 410), the apparatus comprising:
an interface (202) for providing the control signal to the image guided system and for obtaining an optical image of a part of a body; and
a processor (204) configured to:
Obtain information on anatomical features, the information on the anatomical features being based on anatomical features identified within a pre-operative three-dimensional scan of the part of the body,
Generate the control signal based on a match between the anatomical features of the information on the anatomical features and corresponding anatomical features visible within the optical image of the part of the body, wherein the control signal indicates an alignment between the optical image and the pre-operative three-dimensional scan, and
Provide the control signal to the image guided system via the interface.

6. The apparatus according to claim 5, wherein the optical image is part of a video stream of the part of the body, wherein the control signal is updated based on updates to the optical image within the video stream of the part of the body.

7. The apparatus according to one of the claims 5 or 6, wherein the processor is configured to identify anatomical features corresponding to the anatomical features of the information on the anatomical features within the optical image, wherein the control signal is generated based on an alignment between the anatomical features of the information on the anatomical features and the anatomical features identified within the optical image.

8. The apparatus according to claim 7, wherein a machine-learning model is used to identify different categories of anatomical features within the optical image, wherein the anatomical features are represented according to the different categories of anatomical features within the information on the anatomical features, wherein the control signal is generated based on an alignment between the anatomical features of the information on the anatomical features of a category of the different categories of anatomical features and the anatomical features of the category identified within the optical image,
and/or wherein the optical image is a multi-spectral optical image, wherein the anatomical features are identified within the optical image using two more spectral bands of the multi-spectral optical image.

9. The apparatus according to one of the claims 5 to 8, wherein the processor is configured to obtain information on a surface structure of a tissue of the part of the body, the information on the surface structure being based on the pre-operative three-dimensional scan of the part of a body, wherein the processor is configured to identify the surface structure of the tissue of the part of the body within the optical image, wherein the control signal is generated based on an alignment between the obtained information on the surface structure and the surface structure identified within the optical image.

10. The apparatus according to claim 9, wherein the processor is configured to identify the surface structure of the tissue of the part of the body within a depth image being aligned with the optical image, wherein the control signal is generated based on an alignment between the obtained information on the surface structure and the surface structure identified within the depth image, wherein the depth image is one of a Time-of-Flight-based depth image, a structured light-based depth image and a depth image of a stereoscopic camera.

11. The apparatus according to one of the claims 5 to 10, wherein the optical image is a microscopic image obtained from a camera of a microscope, wherein the match between the anatomical features of the information on the anatomical features and the corresponding anatomical features visible within the optical image of the part of the body is based on a zoom level of the microscope,
and/or wherein rigid features of the part of the body are used as a starting point in an alignment between the anatomical features of the information on the anatomical features and the corresponding anatomical features visible within the optical image of the part of the body.

12. The apparatus according to one of the claims 5 to 11, wherein the processor is configured to identify anatomical features corresponding to the anatomical features of the information on the anatomical features within an intraoperative scan of the part of the body, the intraoperative scan having a known orientation relative to the optical image, wherein the control signal is generated based on an alignment between the anatomical features of the information on the anatomical features and the anatomical features identified within the intraoperative scan of the part of the body,
and/or wherein the image guided system is suitable for overlaying a visual augmented reality representation of the pre-operative three-dimensional scan over the optical image of the part of the body, wherein the control signal is suitable for aligning the visual augmented reality representation of the pre-operative three-dimensional scan with the optical image based on the anatomical features.

13. A method for processing a pre-operative three-dimensional scan of a part of a body, the method comprising:
Identifying (110) anatomical features within the pre-operative three-dimensional scan of the part of the body, the anatomical features being features that are visible in an optical image of the part of the body, and
Providing (120) information on the anatomical features based on the anatomical features identified within the pre-operative three-dimensional scan of the part of the body.

14. A method for providing a control signal to an image guided system (220; 410), the method comprising:
Obtaining (230) information on anatomical features, the information on the anatomical features being based on anatomical features identified within a pre-operative three-dimensional scan of a part of a body;
Obtaining (240) an optical image of the part of the body;
Generating (250) the control signal based on a match between the anatomical features of the information on the anatomical features and corresponding anatomical features visible within the optical image of the part of the body, wherein the control signal indicates an alignment between the optical image and the pre-operative three-dimensional scan; and
Providing (260) the control signal to the image guided system.

15. A computer program with a program code for performing at least one of the methods according to one of the claims 13 or 14 when the computer program is executed on a processor.
